# EUROPÄISCHE PATENTSCHRIFT

(11) **EP 1 416 843 B1**
(45) Veröffentlichungstag und Bekanntmachung des Hinweises auf die Patenterteilung: **03.12.2008**
(21) Anmeldenummer: 02760106.1
(22) Anmeldetag: 25.07.2002
(51) Int. Cl.: A61B 3/14

(54) **VORRICHTUNG ZUR KONTAKTLOSEN GEWINNUNG VON BILDERN DER KORNEA**
DEVICE FOR OBTAINING IMAGES OF THE CORNEA IN A NON-CONTACT MANNER
DISPOSITIF PERMETTANT D'OBTENIR SANS CONTACT DES IMAGES DE LA CORNEE

(30) Priorität: 09.08.2001 DE 10138158
(43) Veröffentlichungstag der Anmeldung: 12.05.2004
(73) Patentinhaber: Rhine-tec Gesellschaft für Virtuelle Instrumentierung mbH, 47807 Krefeld (DE)
(72) Erfinder: DAHMEN, Norbert, 47918 Tönisvorst (DE); DIMIC, Zdenko, 41179 Mönchengladbach (DE); SCHILLINGS, Peter, 47441 Moers (DE); SIEGLAR, Volker, 41812 Erkelenz (DE); VEHRESCHILD, Dirk, 47809 Krefeld (DE)
(74) Vertreter: Eisenführ, Speiser & Partner
(86) Internationale Anmeldenummer: PCT/DE2002/002735
(87) Internationale Veröffentlichungsnummer: WO 2003/015623

(56) Entgegenhaltungen:
- DE-A- 4 240 583
- US-A- 4 597 650
- US-A- 5 544 252
- US-A- 5 621 488
- US-A- 5 757 463
- PATENT ABSTRACTS OF JAPAN vol. 018, no. 664 (C-1288), 15. Dezember 1994 (1994-12-15) & JP 06 261864 A (TOPCON CORP), 20. September 1994 (1994-09-20)

## Beschreibung

Die Erfindung betrifft eine Vorrichtung zur kontaktlosen Gewinnung eines vergrößerten Bildes eines Ausschnitts einer Endothel-Zellschicht der Kornea eines Auges eines Patienten nach dem Oberbegriff des Patentanspruches 1.

Eine derartige Vorrichtung ist aus US-A-5,621,488 bekannt. Bei der bekannten Vorrichtung wird mit Hilfe einer Spaltlichtquelle die Augenkornea eines Patienten ausgeleuchtet und mittels einer elektronischen Auswerteeinrichtung und Bildwandler wird eine Serie von drei Bildern der Zellschicht aufgenommen und gespeichert. Dabei wird das qualitativ beste Bild aus der Serie der aufgenommenen Bilder vom Benutzer ausgewählt und für die weitere Verarbeitung, insbesondere Zellzählung bereitgestellt. Hierzu werden die Bilder nacheinander aus dem Speicher abgerufen und in zeitlicher Aufeinanderfolge für die Auswahl bildlich wiedergegeben.

Eine aus US-A-5,757,463 bekannte Vorrichtung beinhaltet zur Darstellung eines Übersichtbildes der Kornea einen ersten Bildwandler und zur Darstellung eines vergrößerten Ausschnitt der Endothel-Zellschicht der Kornea einen zweiten Bildwandler. Das Beleuchtungslicht der Spaltlichtquelle wird über einen schwingenden Spiegel auf die Augenkornea gerichtet, um den gewünschten Korneabereich abzutasten. Um das reflektierte Licht am Schwingspiegel vorbeizuleiten, sind zusätzliche optische Mittel erforderlich, wobei zur Erzeugung des vergrößerten Bildes der Endothel-Zellschicht eine zusätzliche Lichtquelle zur Beleuchtung der Augenkornea verwendet wird und das dabei reflektierte Licht als paralleles Strahlenbündel dem zugeordneten Bildwandler zuzuleiten ist.

Bei einer aus der EP 0 643 941 A1 bekannten Vorrichtung wird die Hornhaut mittels einer Abbildungsoptik in vergrößertem Maßstab abgebildet, z.B. auf eine Kamera, ein Okular oder einen Bildwandler. Zur Beleuchtung der Hornhaut wird bei derartigen Verfahren eine Spalteleuchtungsanordnung verwendet, die in einem Winkel zur.optischen Achse der Abbildungsoptik angeordnet ist. Durch die schräge Einstrahlung und die Verwendung einer Spaltlichtquelle ist gewährleistet, dass die starken Reflexe an der Hornhautoberfläche nicht stören. Gegenüber diesen starken Reflexen ist nämlich das Bild der Endothelzellschicht sehr schwach ausgeprägt, da es sich hier um Strukturen mit einem nur geringem Brechzahlunterschied gegenüber den sonstigen Augenstrukturen handelt. Um ein scharfes und kontrastreiches Bild der Endothelzellschicht zu erzielen, ist weiterhin eine sehr genaue Fokussierung der Abbildungsoptik auf die Zellschicht erforderlich. Bereits Verschiebungen des Auges im Mikrometerbereich können zu einer unerwünschten Defokussierung führen. Derartige Verschiebungen lassen sich bei den kontaktlosen Bildgebungsverfahren jedoch aufgrund unkontrollierter Augenbewegungen nicht vermeiden, so dass die Fokussierung ein schwieriges Problem darstellt.

Bei dem aus dem o.g. Dokument bekannten Verfahren wird dieses Problem dadurch gelöst, dass die korrekte Fokussierung durch ein optisches System erkannt wird. Im Moment der korrekten Fokussierung wird eine Blitzlampe in der Spaltbeleuchtungsanordnung ausgelöst und die entstehende Momentaufnahme der Hornhaut festgehalten. Nachteilig bei diesem Ansatz ist der relativ hohe Aufwand an optischen und elektromechanischen Komponenten für die Bestimmung des fokussierten Zustandes sowie der Einsatz einer Blitzlampe, der zu Blendungseffekten des Auges führen kann, für den Patienten unangenehm ist (Erschrecken) sowie die Anordnung wartungsaufwendiger macht. Außerdem muss die Blitzlampe mit Hochspannung versorgt werden, was bei einem im Kopfbereich eingesetzten medizinischen Gerät unter Sicherheitsaspekten unerwünscht ist. Weiterhin können die bei der Gasentladung fließenden Ströme elektromagnetische Störungen anderer Geräte verursachen.

Weiterhin ist bei der bekannten Vorrichtung nachteilig, dass die spätere Auswertung der Bilder in der Regel visuell durch den Arzt bzw. den Bediener des Gerätes durchgeführt wird, so dass eine standardisierte, objektivierte Auswertung nicht gegeben ist. Sollen objektivierbare Kenngrößen wie die Endothel-Zelldichte gewonnen werden, so müssen diese bei den bekannten Verfahren ausgezählt werden, was einen hohen zeitlichen und personellen Aufwand zur Folge hat und auch die Gefahr von Zählfehlern mit sich bringt.

Der vorliegenden Erfindung liegt die Aufgabe zugrunde, eine Vorrichtung der eingangs genannten Art dahingehend zu verbessern, dass diese mit geringerem optischen und elektromechanischen Aufwand sowie schnell und einfach einzusetzen ist, wobei jedwede Belastung des Auges des Patienten vermieden werden soll.

Die Lösung der vorstehend genannten Aufgabe erfolgt mit den Merkmalen des Patentanspruchzs1.

Zweckmäßige Ausgestaltungen der Erfindung ergeben sich aus den Unteransprüchen.

Die korrekte Fokussierung wird dadurch erreicht, dass ausgehend von einer annähernd fokussierten Grundeinstellung eine Sequenz - d.h. eine Bildfolge nach Art eines Films - von Bildern des Endothels (bzw. des Epithels) aufgenommen wird. Da der Kopf und das Auge ständige unkontrollierte Bewegungen ausführen, ist es bei genügender Anzahl der aufgenommenen Bilder hinreichend wahrscheinlich, dass eines oder mehrere der aufgenommenen Bilder ausreichend scharf sind. Im Anschluss an die Aufnahme der Sequenz wird dann das optimale Bild ausgewählt, wozu Bildverarbeitungsmethoden herangezogen werden. Das auf diese Weise erhaltene Bild wird dann weiteren Auswertungen unterzogen, insbesondere hinsichtlich der Zelldichte und der Morphologie der Zellen. Der Vorteil dieser vorgehensweise liegt darin, dass eine aufwendige optische Anordnung zur Bestimmung des Fokus nicht erforderlich ist. Diese Bestimmung kann rein softwaremäßig durchgeführt werden. Dadurch, dass die unkontrollierte Bewegung des Auges ausgenutzt wird, ist auch kein Aktuator erforderlich, mit dem der Fokus computergesteuert verändert werden müsste. Es ist lediglich eine Justierung in eine Grundeinstellung erforderlich, die auch manuell durchgeführt werden kann. Insgesamt sind im Rahmen des erfindungsgemäßen Verfahrens somit Aktuatoren wie z.B. Schrittmotoren nicht zwingend erforderlich, was - sofern die Optik gekapselt und stabil aufgebaut ist - einen nahezu wartungsfreien Aufbau der erfindungsgemäßen Anordnung erlaubt.

Außerdem kann im Rahmen der Erfindung auf eine Blitzlampe verzichtet werden, so dass eine entsprechende Blendgefahr nicht gegeben ist und die vorstehend aufgeführten, mit dem Einsatz einer Blitzlampe verbundenen Nachteile entfallen. So ist im Rahmen der Erfindung keine Hochspannungsversorgung erforderlich, vielmehr kann die Anordnung komplett im Niederspannungsbereich betrieben werden.

Bei der Erfindung sind zwei elektronische Bildwandler (z.B. CCD-Kameras) vorgesehen, wobei der erste ein Übersichtsbild liefert und der zweite Bildwandler über eine Vergrößerungsoptik ein derart vergrößertes Bild bereitstellt, dass die einzelnen Zellen der zu untersuchenden Hornhaut erkennbar sind. Das Übersichtsbild dient zum einen als Justierhilfe, indem bestimmte Merkmale des Übersichtsbildes mit Justiermarken an festen Positionen im Übersichtsbild in Deckung gebracht werden, wie anhand der Zeichnungen näher erläutert werden wird. Zum anderen erlaubt das Übersichtsbild die Bestimmung zusätzlicher Messwerte.

Insbesondere ist in einer bevorzugten Ausführungsform der Erfindung vorgesehen, dass das Auge für das Übersichtsbild zunächst mittels einer IR-Lichtquelle beleuchtet wird. Da somit - bei ansonsten abgedunkelter Messumgebung - das Auge nicht im sichtbaren Bereich beleuchtet wird, bleibt die Pupille weit geöffnet und das Auge wird nicht belastet. Durch Aktivierung einer zusätzlichen, im sichtbaren Spektralbereich betriebenen Lichtquelle schließt sich die Iris. Von diesem Schliessvorgang kann ebenfalls eine Sequenz von Bildern durch den ersten Bildwandler aufgenommen werden. Durch eine rechnergesteuerte Auswertung dieser Bilder mit Bestimmung des zeitabhängigen Pupillendurchmessers kann die Reaktionszeit der Pupille und die Geschwindigkeit des Schließvorganges analysiert werden, woraus Rückschlüsse auf evtl. neurologische Störungen möglich sind. Auf diese Weise können somit mittels eines Gerätes verschiedene Untersuchungen ohne zusätzlichen Aufwand quasi simultan durchgeführt werden.

Zusätzlich zu einer Vorrichtung, die zur Durchführung des vorstehend erläuterten Verfahrens ausgebildet ist, wird weiterhin im Rahmen der vorliegenden Erfindung eine Vorrichtung vorgeschlagen, bei der der erste und der zweite Bildwandler über eine Strahlteilereinrichtung an dieselbe Abbildungsoptik gekoppelt sind, so dass die Abbildung für beide Bildwandler entlang derselben optischen Achse erfolgt. Damit wird eine exakte und parallaxenfehlerfreie Ausrichtung der Bilder des ersten und des zweiten Bildwandlers sichergestellt, wodurch die Justierung und Auswertung der Bilder erleichtert wird. Bei Einsatz entsprechend hochauflösender Sensoren ist es auch denkbar, dass der erste und der zweite Bildwandler in eine Chipfläche integriert sind, wobei die Vergrößerung des Übersichtsbildes in diesem Falle mit Hilfe der Software erfolgen kann.

Um verschiedene Bereiche des Endothels abbilden zu können, kann die Spaltlichtquelle auf einem Schwenkarm um die optische Achse herum schwenkbar ausgebildet sein.

Die erfindungsgemäße Vorrichtung ist im Vergleich zu bekannten Vorrichtungen erheblich weniger aufwendig und daher erheblich preiswerter und wartungsärmer. Da die Messung kontaktlos und ohne Belastung des Auges durch hohe Lichtintensitäten erfolgen kann, können mit der erfindungsgemäßen Vorrichtung Vorsorgeuntersuchungen der Hornhaut ohne Risiken durchgeführt werden, wobei diese Untersuchungen auch von nichtmedizinischen Bedienpersonen - z.B. Augenoptikern - durchgeführt werden können. Weiterhin können die Untersuchungen und die nachfolgenden Auswertungen im Rahmen der Erfindung sehr schnell durchgeführt werden.

Die Erfindung wird nachfolgend anhand der Zeichnungen beispielhaft näher erläutert. Es zeigen:
- Figur 1: eine schematische Ansicht des optischen Aufbaus eines Ausführungsbeispiels der erfindungsgemäßen Vorrichtung;
- Figuren 2a und 2b: schematische Ansichten des Auges zur Erläuterung des Justiervorganges;
- Figur 3: ein schematisches Blockdiagramm eines Ausführungs-beispiels des erfindungsgemäßen Verfahrens; und
- Figur 4: eine Übersicht über die Hardwareeinrichtungen und die Softwaremodule, die bei einer erfindungsgemä-ßen Vorrichtung eingesetzt werden.

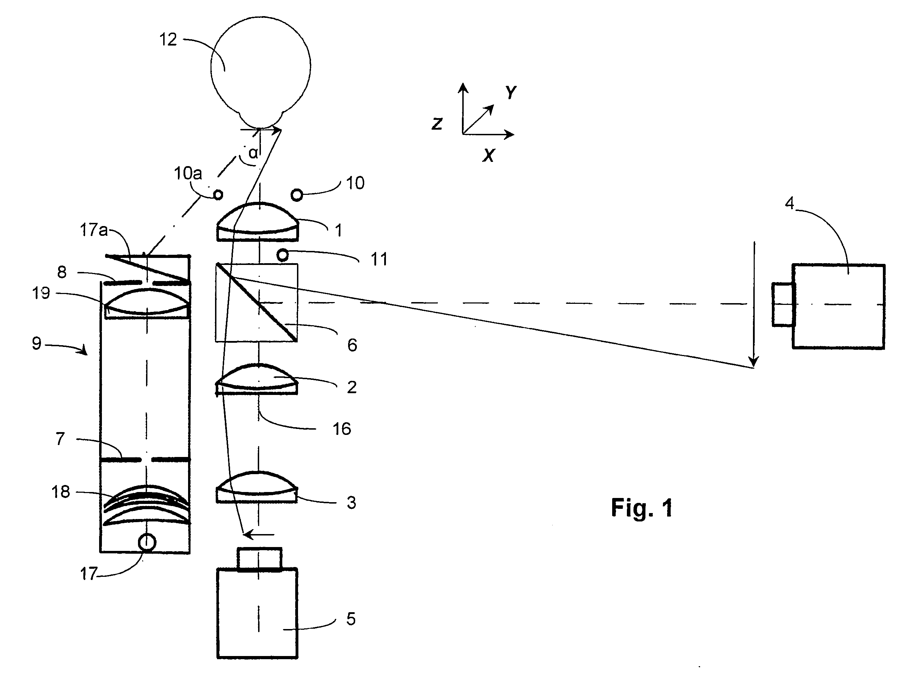

In Figur 1 ist eine multifunktionale optoelektronische Vorrichtung für kontaktlose Untersuchungen eines schematisch angedeuteten Auges 12 eines Patienten dargestellt. Die Vorrichtung ist mittels einer (nicht dargestellten) Verstelleinrichtung in ihrer Gesamtheit in X-, Y- und Z-Richtung justierbar. Die Vorrichtung weist weiterhin eine Abbildungsoptik auf, die mittels der Konvexlinsen 1, 2 und 3, die entlang einer optischen Achse 16 angeordnet sind, im fokussierten Zustand das Bild des Endothels bzw. (je nach eingestelltem Fokus) des Epithels des Auges 12 mit einem Abildungsmaßstab, der nahe bei 1 : 1 liegt, auf einen ersten Bildwandler 5 abbilden, um ein übersichtsbild des Auges zu erhalten. Der als CCD-Sensor ausgebildete Bildwandler 5 ist mit einer (nicht dargestellten) Auswertungseinheit verbunden, die aus einer Framegrabberkarte zur Umwandlung der Kamerasignale in digitale Bildsignale, einem handelsüblichen Computer mit üblicher Peripherie (Tastatur, Maus, Monitor, evtl. Drucker) und mit einem Interface zur Framegrabberkarte und mit einem Interface zur Interaktion mit der Messhardware besteht. Das aktuelle Bildsignal des Bildwandlers 5 kann über den Computer auf dem Monitor wiedergegeben werden. Ein zweiter, ebenfalls über eine Framegrabberkarte an die Auswertungseinheit angeschlossener Bildwandler 4 erhält ein über eine zwischen den Linsen 1 und 2 angeordnete Teileroptik 6 ausgekoppeltes Bild der abzubildenden Zellschicht der Hornhaut. Die Teileroptik 6 enthält einen teildurchlässigen Spiegel, der die Strahlen beispielsweise im Verhältnis 90 (Bildwandler 4) zu 10 (Bildwandler 5) teilt. Durch die Wahl einer relativ großen Bildweite ist der Abbildungsmaßstab für den zweiten Bildwandler stark vergrößert, so dass in dem Bild des Bildwandlers 4 die Zellstrukturen der Hornhaut sichtbar werden. Beide Bildwandler 4 und 5 nehmen das Auge entlang derselben optischen Achse 16 auf und sind derart angeordnet, dass das Bild auf beiden Bildwandlern bei korrekter Einstellung der Gegenstandsweite durch Veränderung des Abstandes der Anordnung zum Auge gleichzeitig scharf abgebildet wird. Bevorzugt wird auf dem an den Computer angeschlossenen Monitor das vergrößerte Bild des zweiten Bildwandlers 4 dargestellt, wobei das Übersichtsbild des ersten Bildwandlers 5 an einer vorgegebenen Stelle in einen Ausschnitt des Monitorbildes eingeblendet wird.

Weiterhin ist eine Spaltbeleuchtungsanordnung 9 vorgesehen, die eine LED 17 als Lichtquelle, zwei Spaltblenden 7 und 8 (Spaltdurchmesser vergrößert dargestellt), eine Abbildungsoptik mit Linsen 18, 19 sowie ein Umlenkprisma 17a aufweist. Das Bild der Spaltblende 7 wird in einem Winkel α von etwa 45° zur optischen Achse 16 auf die abzubildende Zellschicht des Auges 12 abgebildet. Die gesamte Spaltbeleuchtungsanordnung 9 ist auf einer Kreisbahn um die optische Achse 16 schwenkbar angeordnet, so dass verschiedene Bereiche der Hornhaut beobachtet werden können. Aufgrund der Geometrie der Augenhöhle sind insbesondere die in der Zeichnung dargestellte Position der Spaltbeleuchtungsanordnung 9 sowie eine hinsichtlich der optischen Achse 16 gespiegelte Anordnung auf der rechten Seite der optischen Achse möglich. Damit in dieser letzteren Position der Lichtweg auf den Bildwandler 4 nicht gestört wird, kann dieser Bildwandler auch um 90° gedreht oberhalb oder unterhalb der Papierebene angeordnet werden, was gleichzeitig einen kompakteren Aufbau der Vorrichtung ermöglicht. Eine Fixierlichtquelle 11, die als kleine, nur schwach leuchtende LED ausgebildet ist, wird vom Patienten durch das zu untersuchende Auge fixiert, um den Blick in eine definierte Richtung zu lenken und unkontrollierte Augapfelbewegungen zu reduzieren.

Bevorzugt ist die Fixierlichtquelle - bezogen auf das Blickfeld des Patienten - oberhalb der optischen Achse angeordnet, wodurch in vorteilhafter Weise aufgrund der Motorik des Augenlides das Auge intuitiv weit geöffnet ist.

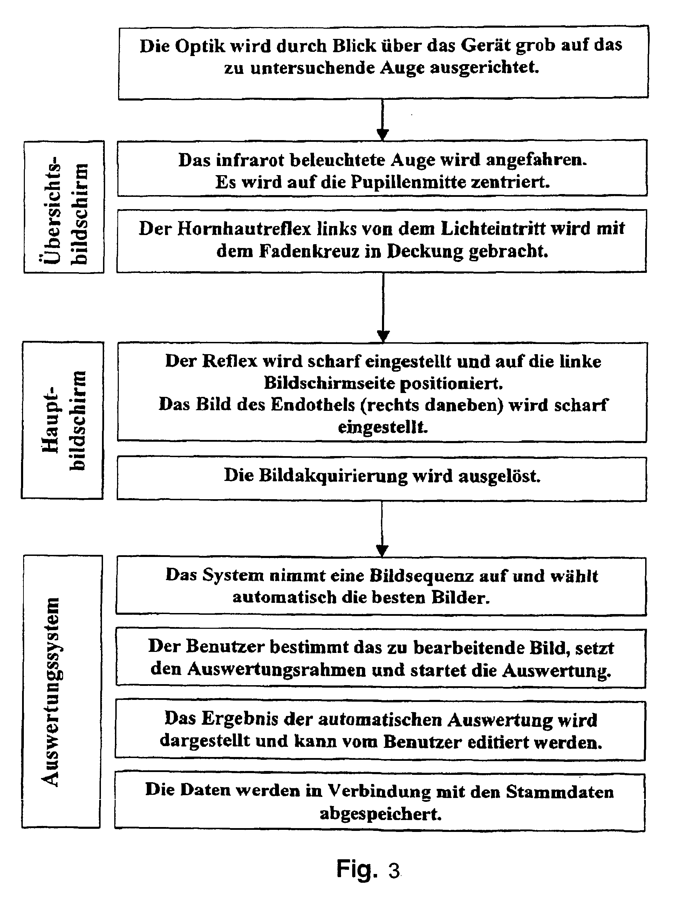

Der Ablauf der Bildgewinnung bei der dargestellten Vorrichtung ist wie folgt (vgl. auch das Blockdiagramm in Figur 3):

Zunächst muss die Vorrichtung auf das abzubildende Auge ausgerichtet bzw. justiert werden, da unterschiedliche Augen bezüglich ihrer Geometrie sowie der jeweiligen Kopfposition Abweichungen aufweisen. Ein möglicher Ablauf eines derartigen Justiervorganges ist wie folgt:

Die Vorrichtung wird zunächst grob durch einen Blick über das Gerät auf das zu untersuchende Auge ausgerichtet (Schritt 30). Dann wird das Auge 12 zunächst mittels einer Infrarot-Leuchtdiode 10 beleuchtet, die seitlich der Linse 1 an der dem Auge 12 zugewandten Seite angeordnet ist. Es können auch mehrere IR-Leuchtdioden vorgesehen sein. Auf dem Bildschirm wird das Bild des infrarotempfindlichen Bildwandlers 5 dargestellt, das ein Übersichtsbild des Auges darstellt. In die Bildschirmwiedergabe des Übersichtsbildes wird eine Justiermarke an einer festen Position in der Bildmitte eingeblendet. Diese Justiermarke wird durch Verschiebung der Vorrichtung in X- und Y-Richtung mit der Pupillenmitte zwecks Durchführung einer weiteren Grobausrichtung in Übereinstimmung gebracht (Schritt 31). Diese und die nachfolgenden Justiervorgänge erfolgen bevorzugt manuell, können jedoch auch bei Vorhandensein entsprechender Stellmotoren für die Positionierung der Vorrichtung automatisch durch eine Auswertung und Verarbeitung der Bilder der Bildwandler 4, 5 durchgeführt werden.

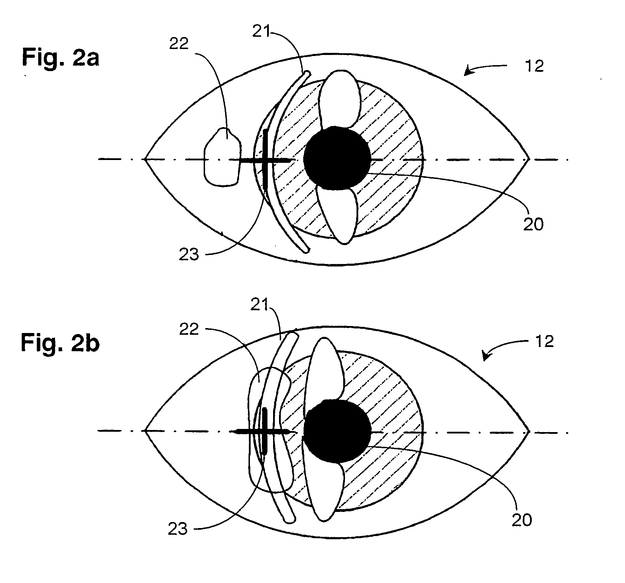

Zur weiteren Justierung wird die Spaltbeleuchtungsanordnung 9 eingeschaltet. Wie aus Figur 2a ersichtlich, tritt das Licht der Spaltbeleuchtungsanordnung 9 in dem in diesem Stadium erreichten Justierzustand in einem sichelförmigen Bereich 21 links von der optischen Achse 16 auf das Auge 12. Dieser Bereich 21 und der Einfallswinkel des Lichts der Spaltbeleuchtungsanordnung 9 sind derart gewählt, dass das Bild der Endothelzellschicht auf der Rückseite der Hornhaut bei korrekt justierter Anordnung direkt rechts neben dem epithelseitigen Reflex 22 der Spaltlichtquelle 9 sichtbar wird. Nur in dieser Position ist eine befriedigende Abbildung möglich, in der einerseits die Endothelzellschicht durch die Spaltlichtquelle beleuchtet und andererseits das Bild nicht durch den Reflex an der Hornhautvorderseite überstrahlt wird. Um diese spezifischen Abbildungsbedingungen zu erhalten, muss der Mittelpunkt des Reflexes 22 mit der im Übersichtsbild eingeblendeten Justiermarke 23 in Deckung gebracht werden (Schritt 32). Auf diese Weise wird die in Figur 2b dargestellte, korrekt justierte Position erreicht, bei der der Einstrahlbereich 21, der Reflex 22 und die Justiermarke 23 übereinander liegen.

Nachdem die Anordnung in diesem Zustand bereits derart positioniert ist, dass der zweite Bildwandler 4 den gewünschten Bereich des Auges 12 abbilden kann, erfolgt die weitere Justierung anhand des vergrößerten Bildes des zweiten Bildwandlers 4. Zunächst erfolgt eine Feinjustierung in X-, Y-und Z-Richtung, so dass der Reflex 22 in der linken Bildhälfte des vergrößerten Bildes sichtbar wird und scharf dargestellt wird. In der rechten Bildhälfte, angrenzend an den Reflex 22, wird dann das Bild der Endothelzellschicht sichtbar, das durch eine Feinabstimmung der Z-Position möglichst scharf gestellt wird (Schritt 33).

Durch die unvermeidliche Bewegung des Auges ist diese Scharfeinstellung jedoch nur näherungsweise und für einen kurzen Augenblick möglich. Wird ein entsprechender Zustand erreicht, so wird - vorzugsweise durch Druck auf einen Auslöseknopf direkt an der Vorrichtung - eine Bildakquirierung ausgelöst (Schritt 34).

Daraufhin wird durch den Computer eine Serie von beispielsweise fünfzig Bildern des Bildwandlers 4 gespeichert. Dabei ist es sehr wahrscheinlich, dass ein oder mehrere der aufgenommenen Bilder hinreichend scharf sind. Die Bilder werden zunächst im Speicher des Computers (d.h. im RAM oder auf der Festplatte) abgelegt und erst nach Aufnahme der vollständigen Bildsequenz analysiert. Alternativ ist es auch denkbar, bereits während der Aufnahme die Bilder zu analysieren und von vornherein als ungeeignet erkannte Bilder nicht zu speichern.

Anschließend erfolgt die computerunterstützte Auswahl der gespeicherten Bilder. Dazu werden bevorzugt mehrere Kriterien berechnet, anhand derer die Qualität der Bilder quantifiziert werden kann. Dies kann der maximale oder mittlere Kontrast des jeweiligen Einzelbildes sein, der bei einem scharfen Bild größer ist als bei einem unscharfen. Vorzugsweise wird mittels einer Fast-Fourier-Transformation die diskrete Fourier-Transformierte des Bildes berechnet, die ein Maß für das Auftreten periodisch wiederkehrender Strukturen in den Bildern darstellt. Bei einer scharfen Darstellung stellen die Endothelzellen eine periodische Struktur mit einem vorgegebenen mittleren Abstand dar, wodurch sich hohe Amplituden in der Fourier-Darstellung ergeben. Durch Berechnung einer Größe wie der mittleren quadratischen Abweichung zu der Fourier-Transformierten eines Referenzbildes oder eine ähnliche Operation kann auf diese Weise die Qualität der Einzelbilder bewertet werden. Bevorzugt werden die vorstehend genannten Operationen nur auf den Bildbereich angewandt, in denen das Bild der Endothelzellschicht vermutet wird. Von den auf diese Weise bewerteten Einzelbildern werden eine bestimmte Anzahl mit der jeweils höchsten Wertung (z.B. fünf Aufnahmen) ausgewählt (Schritt 35) und dem Benutzer auf dem Bildschirm angezeigt, wobei dann der Benutzer die endgültige Auswahl der optimalen Aufnahme vornimmt (Schritt 36). Sollte keine der Aufnahmen der Sequenz eine ausreichende Qualität aufweisen, was z.B. passieren kann, wenn die Aufnahmesequenz in einem Moment gestartet wurde, in dem die Augenposition aufgrund der unkontrollierten Bewegungen sehr weit von der mittleren Augenposition entfernt ist, wird die Sequenz erneut aufgenommen.

Das auf diese Weise gewonnene Bild kann zusammen mit den Patientendaten dauerhaft gespeichert und ausgedruckt werden.

Bevorzugt werden aus dem Bild bestimmte wesentliche Kennzahlen bestimmt. Die wichtigste Kennzahl ist die Endothel-Zelldichte. Konventionell wird diese Zelldichte bestimmt, indem eine Photographie des Endothels angefertigt und die Anzahl der Zellen in einem Rechteck vorgegebener Größe manuell ausgezählt wird, was mit einem enormen Zeitaufwand verbunden ist (sog. Fixed-Frame-Methode).

Im Rahmen der vorliegenden Erfindung kann die Zelldichtebestimmung nahezu vollautomatisch erfolgen. Zunächst wird bevorzugt durch den Benutzer mittels Mausbedienung ein repräsentativer Bildbereich mit einer variablen Fläche eingerahmt, in dem die Zahl der Zellen bestimmt werden soll (Schritt 36). Der Rahmen wird so positioniert, dass die in dem Rahmen enthaltenen Zellen hinsichtlich ihrer Dichte und Morphologie möglichst repräsentativ für die übrigen Zellen sind. Innerhalb des Rahmens erfolgt die computergestützte Erkennung der einzelnen Zellen und daran anschließend die computergestützte Auswertung mittels Bildverarbeitung.

Das Ergebnis der Erkennung wird auf dem Bildschirm dargestellt. Fehlerhaft erkannte Objekte können durch Markierung auf dem Bildschirm nachkorrigiert werden (Schritt 37). Schließlich erfolgt die Zählung der Anzahl der klassifizierten Objekte in dem Bereich und aus einer Division durch die Fläche des Bereichs die Dichtebestimmung.

Weiterhin werden die erkannten Objekte anhand der vorstehend bestimmten Größen hinsichtlich ihrer Morphologie und der Zellflächen klassifiziert. Dazu werden die prozentualen Anteile der Zellen, die eine 3-, 4-, 5-, 6-, 7- oder mehreckige Form aufweisen, bestimmt. Idealerweise sollten sämtliche Zellen eine hexagonale Form aufweisen. Die so gewonnenen Daten werden am Bildschirm dargestellt und können anschließend archiviert werden (Schritt 38).

Weiterhin kann mit der erfindungsgemäßen Anordnung der Pupillenreflex vermessen werden. Dazu ist bevorzugt eine zusätzliche, im sichtbaren Spektralbereich emittierende Lichtquelle 10a vor dem Auge angeordnet. Grundsätzlich kann jedoch auch die Spaltbeleuchtungsanordnung 9 als derartige Lichtquelle dienen. Wird die zusätzliche Lichtquelle 10a bei vorher unbeleuchtetem bzw. nur mit Infrarotlicht beleuchtetem Auge 12 eingeschaltet, so verengt sich die Pupille 20 durch den dadurch entstehenden Lichtreiz. Diese Reaktion kann computergestützt verfolgt werden, indem der Einschaltzeitpunkt der Lichtquelle 10a erfasst und mittels Bildverarbeitung der Pupillendurchmesser in dem Übersichtsbild in regelmäßigen Zeitabständen (z.B. 10 mal pro Sekunde) ausgemessen wird. Die entsprechenden Algorithmen zur Erkennung des Pupillendurchmessers sind für den Fachmann ohne weiteres ersichtlich. Der so erhaltene zeitliche Verlauf des Pupillendurchmessers kann dann in einem Diagramm auf dem Bildschirm dargestellt und mit entsprechenden Normwerten verglichen werden, um Auffälligkeiten des Pupillenreflexes festzustellen. Die gewonnenen Messdaten können weiterhin mittels des Computers archiviert werden.

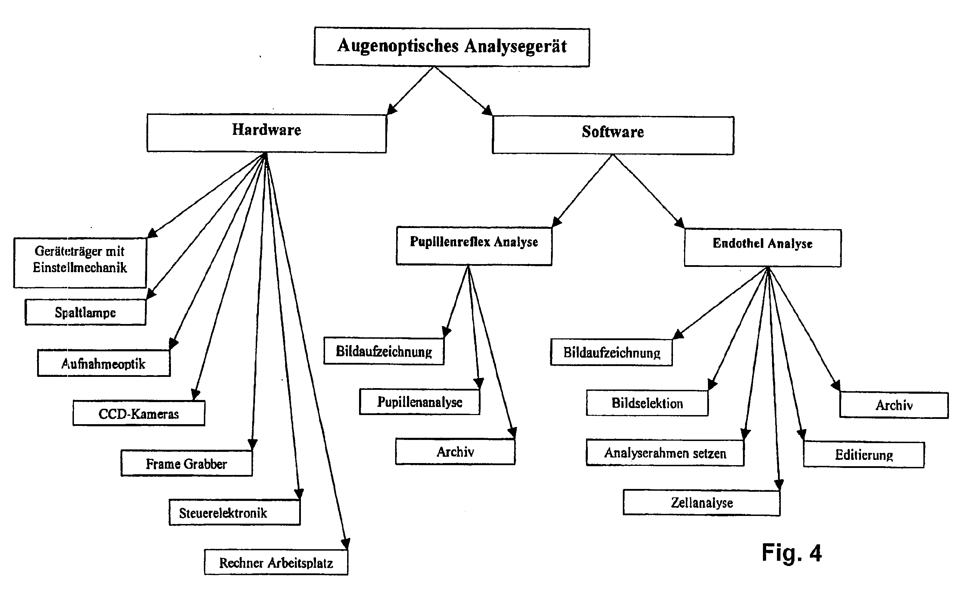

Figur 4 zeigt eine Übersicht über die verschiedenen Hard-und Softwaremodule. Die Software ist gemäß den zwei dargestellten Blöcken in die Funktionsblöcke Endothelanalyse und Pupillenanalyse aufgeteilt, die jeweils in einzelne Unterblöcke unterteilt sind.

## Patentansprüche

1. Vorrichtung zur kontaktlosen Gewinnung eines vergrößerten Bildes eines Ausschnitts der Endothel-Zellschicht der Kornea eines Auges (12) eines Patienten mit einer Spaltbeleuchtungsanordnung (9), einer Abbildungsoptik zum Abbilden eines mittels der Spaltbeleuchtungsanordnung (9) ausgeleuchteten Ausschnitts der abzubildenden Korneazellschicht auf wenigstens einen mit einer elektronischen Auswertungseinheit in Verbindung stehenden elektronischen Bildwandler (4, 5), einer Einrichtung zur Justierung der Abbildungsoptik in eine Grundeinstellung, in der die abzubildende Zellschicht näherungsweise scharf und vergrößert auf den Bildwandler (4, 5) abgebildet ist, wobei die Auswertungseinheit ausgebildet ist zur Aufnahme und Speicherung einer Sequenz von Bildern der Zellschicht, während sich die Lage des Auges (12) und die Fokussierung durch unkontrollierte Bewegungen des Auges verändert und zur Auswahl des hinsichtlich Schärfe und/oder Kontrast qualitativ besten Bildes aus der Serie der aufgenommenen Bilder sowie Bereitstellung dieses Bildes zur weiteren Verarbeitung und einem Bildschirm zur Bildwiedergabe, **dadurch gekennzeichnet, dass** die Auswerteeinrichtung ferner für eine Bildauswertung ausgebildet ist zur Auswahl einer vorgegebenen Anzahl der best analysierten Bilder der Sequenz - vorzugsweise etwa fünf -, wobei die ausgewählten Bilder vollständig oder in einem vorgegebenen Teilbereich jeweils maximalen oder mittleren Kontrast aufweisen und/oder deren zweidimensional Fourier-Transformierte die Fourier-Transformierte einer optimalen Referenzaufnahme am besten approximieren und dass der Bildschirm zur Darstellung der ausgewählten Bilder für eine durch den Benutzer anhand einer visuellen Begutachtung zu treffende endgültige Auswahl des weiter zu verarbeitenden Bildes ausgebildet ist.

2. Vorrichtung nach Anspruch 1, **dadurch gekennzeichnet, dass** die Auswerteeinrichtung zur Zelldichtebestimmung in einem ausgewählten Bereich der abgebildeten Zellschicht ausgebildet ist.

3. Vorrichtung nach Anspruch 1 oder 2, **dadurch gekennzeichnet, dass** die Auswerteeinrichtung für die Zellmorphologiebestimmung in einem ausgewählten Bereich der abgebildeten Zellschicht ausgebildet ist.

4. Vorrichtung nach einem der Ansprüche 1 bis 3, **dadurch gekennzeichnet, dass** ein erster Bildwandler (5) zur Erzeugung eines Übersichtbildes der Kornea und ein zweiter Bildwandler (4) zur Erzeugung eines vergrößerten Bildes entlang derselben optischen Achse (16) vorgesehen ist.

5. Vorrichtung nach Anspruch 4, **dadurch gekennzeichnet, dass** der erste (5) und der zweite Bildwandler (4) über eine Strahlteilereinrichtung (6) an dieselbe Abbildungsoptik gekoppelt sind, sodass die Abbildung für beide Bildwandler entlang derselben optischen Achse (16) erfolgt.

6. Vorrichtung nach Anspruch 4, **dadurch gekennzeichnet, dass** der erste und zweite Bildwandler durch ein einziges bilderzeugendes Halbleiterelement gebildet werden, wobei das vergrößerte Bild und das Übersichtsbild softwaremäßig aus dem Bildsignal des Halbleiterelements erzeugt werden.

7. Vorrichtung nach einem der Ansprüche 1 bis 6, **dadurch gekennzeichnet, dass** zur Ausleuchtung des Auges (12) für die Gewinnung des Übersichtbildes eine IR-Lichtquelle (10) vorgesehen ist, wobei der erste Bildwandler (5) im Infrarotbereich empfindlich ist.

8. Vorrichtung nach einem der Ansprüche 1 bis 7, **dadurch gekennzeichnet, dass** die Spaltlichtquelle (9) schwenkbar um die optische Achse (16) angeordnet ist.

9. Vorrichtung nach einem der Ansprüche 1 bis 8, **dadurch gekennzeichnet, dass** eine Fixierlichtquelle (11) vorgesehen ist, die während des Justier-und/oder Messvorganges im Blickfeld des Patienten sichtbar ist, um unkontrollierte Augapfelbewegungen zu reduzieren, wobei die Fixierlichtquelle im Blickfeld oberhalb der optischen Achse sichtbar ist.

10. Vorrichtung nach Anspruch 1, **dadurch gekennzeichnet, dass** zur Beleuchtung des Auges eine Lichtquelle (10a) im sichtbaren Spektralbereich vorgesehen ist und die Auswertungseinheit ausgebildet ist zur Ermittlung einer auftretenden Verengung der Pupille (20) des Auges und deren zeitlichen Verlaufs durch Verarbeitung einer vom ersten Bildwandler (5) erfassten Bildsequenz.

11. Vorrichtung nach einem der Ansprüche 1 bis 10, **dadurch gekennzeichnet, dass** die Auswerteeinrichtung zur Aufnahme und Speicherung von etwa 50 Bildern in einem Zeitraum von etwa 2 Sekunden ausgebildet ist.

## Claims

1. Apparatus for contactless retrieval of a magnified image of a section of the endothelial layer of cells of the cornea of an eye (12) of a patient, comprising a slit illumination assembly (9), imaging optics for imaging a section of said layer of cornea cells to be imaged, which section is illuminated by said slit illumination assembly (9), onto at least one electronic image converter (4, 5) connected to an electronic evaluation unit, means for adjusting the imaging optics to a basic setting in which said layer of cells to be imaged is imaged onto said image converter (4, 5) approximately in focus and with magnification, wherein said evaluation unit is arranged to receive and store a sequence of images of said layer of cells while a position of the eye (12) and focusing are varying due to uncontrolled movements of the eye, and to select from a series of received images an image having best quality in sharpness and/or contrast and to provide this image for processing, and a display for image rendering, **characterized in that** said evaluation unit is further arranged for image evaluation to select from said sequence a predetermined number of - preferably five - images evaluated as the best, wherein said selected images have maximum or medium contrast in whole or in a predetermined portion thereof respectively, and/or the two dimensional Fourier transform thereof approximates at most the Fourier transform of an optimal reference image, and **in that** said display is arranged to present said selected images for a final selection of an image to be processed made by the user on the basis of a visually appraisal.

2. Apparatus as claimed in claim 1, **characterized in that** said evaluation unit is arranged to determine a cell density in a selected area of the imaged layer of cells.

3. Apparatus as claimed in claim 1 or 2, **characterized in that** said evaluation unit is arranged to determine a cell morphology in a selected area of the imaged layer of cells.

4. Apparatus as claimed in any one of claims 1 to 3, **characterized in that** a first image converter (5) for generating an overview image of the cornea and a second image converter (4) for generating a magnified image along the same optical axis (16) are provided.

5. Apparatus as claimed in claim 4, **characterized in that** said first (5) and second (4) image converters are coupled through a beam splitter device (6) to the same imaging optics, so that imaging is performed along the same optical axis (16) for both image converters.

6. Apparatus as claimed in claim 4, **characterized in that** said first and second image converters are formed by a single image forming semiconductor element, wherein said magnified image and said overview image are generated from an image signal of said semiconductor element by software.

7. Apparatus as claimed in any one of claims 1 to 6, **characterized in that** a IR light source (10) is provided for illuminating the eye (12) to retrieve said overview image, wherein said first image converter (5) is sensitive in the infra red range.

8. Apparatus as claimed in any one of claims 1 to 7, **characterized in that** said slit light source (9) is arranged to be rotatable around the optical axis (16).

9. Apparatus as claimed in any one of claims 1 to 8, **characterized in that** a fixing light source (11) is provided which is visible in the visual field of the patient during adjusting and/or measuring in order to reduce uncontrolled eyeball movements, wherein said fixing light source is visible in the visual field above the optical axis.

10. Apparatus as claimed in claim 1, **characterized in that** a light source (10a) within the visible spectral range is provided for illuminating the eye, and **in that** said evaluation unit is arranged to determine an occurring contraction of the pupil (20) of the eye and the temporal course thereof by processing an image sequence detected by the first image converter (5).

11. Apparatus as claimed in any one of claims 1 to 10, **characterized in that** said evaluation unit is arranged to receive and store about 50 images in a period of about 2 seconds.

## Revendications

1. Installation permettant d'obtenir sans contact une image agrandie d'une partie de la couche de cellules d'enthothélium de la cornée d'un oeil (12) d'un patient, comprenant un dispositif (9) d'éclairage de fente, une optique de reproduction pour reproduire une partie, éclairée par le dispositif (9) d'éclairage de fente, de la couche de cellules de cornée à reproduire sur au moins un transformateur (4, 5) électronique d'images en liaison avec une unité électronique d'exploitation, un dispositif d'ajustement de l'optique de reproduction pour la mettre dans un réglage de base dans lequel la couche de cellules à reproduire est reproduite approximativement d'une manière nette et agrandie sur le transformateur (4, 5) d'images dans lequel l'unité d'exploitation est constituée pour enregistrer et mémoriser une séquence d'images de la couche de cellules tandis que la position de l'oeil (12) et la focalisation sont modifiées par des mouvements incontrôlés de l'oeil et pour choisir l'image qualitativement la meilleure du point de vue de la netteté et/ou du contraste dans la série des images enregistrées, ainsi que, pour mettre à disposition cette image en vue du traitement ultérieur, un écran de restitution de l'image, **caractérisée en ce que** le dispositif d'exploitation est constitué en outre pour une exploitation de l'image pour sélectionner un nombre prescrit des images le mieux analysées de la séquence - de préférence environ cinq - les images choisies ayant entièrement, ou dans une sous-partie prescrite, respectivement un contraste maximum ou moyen et/ou leur transformée de Fourier en deux dimensions se rapprochant au mieux de la transformée de Fourier d'un enregistrement de référence le meilleur possible et **en ce que** l'écran est constitué pour représenter les images choisies pour un choix final de l'image à traiter davantage, choix à décider par l'utilisateur au moyen d'une expertise visuelle.

2. Installation suivant la revendication 1, **caractérisée en ce que** le dispositif d'exploitation est constitué pour la détermination de la densité de cellules dans une partie choisie de la couche de cellules reproduite.

3. Installation suivant la revendication 1 ou 2, **caractérisée en ce que** le dispositif d'exploitation est constitué pour la détermination de la morphologie des cellules dans une partie choisie de la couche de cellules reproduite.

4. Installation suivant l'une des revendications 1 à 3, **caractérisée en ce qu'**il est prévu un premier transformateur (5) d'images pour la production d'une image d'ensemble de la cornée et un deuxième transformateur (4) d'images pour la production d'une image agrandie le long du même axe (16) optique.

5. Installation suivant la revendication 4, **caractérisée en ce que** le premier (5) et le deuxième (4) transformateurs d'images sont couplés par un dispositif (16) de division de faisceau à la même optique de reproduction de sorte que la reproduction s'effectue pour les deux transformateurs d'images le long du même axe (16) optique.

6. Installation suivant la revendication 4, **caractérisée en ce que** le premier et le deuxième transformateurs d'images sont formés par un élément à semi-conducteurs unique formant une image, l'image agrandie et l'image d'ensemble étant produites d'une façon logicielle à partir du signal d'images de l'élément à semi-conducteurs.

7. Installation suivant l'une des revendications 1 à 6, **caractérisée en ce que** pour éclairer l'oeil (12) pour l'obtention de l'image d'ensemble, il est prévu une source (10) lumineuse IR, le premier transformateur (5) d'images étant sensible dans le domaine infrarouge.

8. Installation suivant l'une des revendications 1 à 7, **caractérisée en ce que** la source (9) lumineuse de fente est montée pivotante autour de l'axe (16) optique.

9. Installation suivant l'une des revendications 1 à 8, **caractérisée en ce qu'**il est prévu une source (11) de lumière fixe qui, pendant l'opération de réglage et/ou de mesure, est visible dans le champ de vision du patient pour réduire des mouvements incontrôlés du globe oculaire, la source lumineuse fixe étant visible dans le champ de vision au-dessus de l'axe optique.

10. Installation suivant la revendication 1, **caractérisée en ce que,** pour éclairer l'oeil, il est prévu une source (10) lumineuse dans le domaine du spectre visible et l'unité d'exploitation est constituée pour déterminer qu'il se produit un rétrécissement de la pupille (20) de l'oeil et sa courbe dans le temps par traitement d'une séquence d'images détectée par le premier transformateur (5) d'images.

11. Installation suivant l'une des revendications 1 à 10, **caractérisée en ce que** le dispositif d'exploitation est constitué pour l'enregistrement et la mémorisation d'environ 50 images dans un laps de temps d'environ 2 secondes.
